(19) Europäisches Patentamt
European Patent Office
Office européen des brevets

(11) Veröffentlichungsnummer: **0 103 797**
**B1**

(12) # EUROPÄISCHE PATENTSCHRIFT

(45) Veröffentlichungstag der Patentschrift: **26.11.86**

(21) Anmeldenummer: **83108716.8**

(22) Anmeldetag: **05.09.83**

(51) Int. Cl.⁴: **C 07 D 498/06**, A 01 N 43/90 // C07D215/28 ,(C07D498/06, 265:00, 221:00)

(54) **Substituierte Benzoxazin-Derivate.**

(30) Priorität: **17.09.82 DE 3234529**

(43) Veröffentlichungstag der Anmeldung: **28.03.84 Patentblatt 84/13**

(45) Bekanntmachung des Hinweises auf die Patenterteilung: **26.11.86 Patentblatt 86/48**

(84) Benannte Vertragsstaaten: **AT BE CH DE FR GB IT LI NL**

(56) Entgegenhaltungen: **US-A-4 133 954**

**Die Akte enthält technische Angaben, die nach dem Eingang der Anmeldung eingereicht wurden und die nicht in dieser Patentschrift enthalten sind.**

(73) Patentinhaber: **BAYER AG, Konzernverwaltung RP Patentabteilung, D-5090 Leverkusen 1 Bayerwerk (DE)**

(72) Erfinder: **Naumann, Klaus, Dr., Richard- Wagner- Strasse 9, D-5090 Leverkusen 1 (DE)**
Erfinder: **Scheinpflug, Hans, Dr., Am Thelenhof 15, D-5090 Leverkusen 1 (DE)**
Erfinder: **Rosslenbroich, Hans- Jürgen, Dr., Rembrandtstrasse 20, D-4019 Monheim (DE)**
Erfinder: **Paul, Volker, Dr., Windmühlenweg 25, D-4770 Soest (DE)**

**EP 0 103 797 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1986

**Beschreibung**

Die Erfindung betrifft substituierte Benzoxazin-Derivate, mehrere Verfahren zu ihrer Herstellung sowie ihre Verwendung als Pflanzenschutzmittel.

Es ist bereits bekannt, daß bestimmte substituierte bzw. unsubstituierte Benzoxazinone, wie z.B. 4,5-Trimethylenbenzoxazin-3-on, 6-Methyl- und 6-Ethyl-4,5-tri-methylen-benzoxazin-3-on, fungizide Eigenschaften besitzen (vgl. US-PS 4 133 954). Die Wirkung dieser Verbindungen ist jedoch insbesondere bei niedrigen Aufwandmengen und -konzentrationen nicht immer voll befriedigend. Die Herstellung von weiteren Benzoxazinon-Derivaten, wie z.B. 6-Butyl- oder 6-Acetyl-4,5-tri-methylen-1,4-benzoxazin-3-on, ist bekannt (vgl. C.R. Acad. Sc. Paris, Ser. C 1970, 270, 495-501). Über eine Wirkung auf dem Pflanzenschutzgebiet gibt es keine Angaben.

Es wurden neue substituierte Benzoxazin-Derivate der allgemeinen Formel (I)

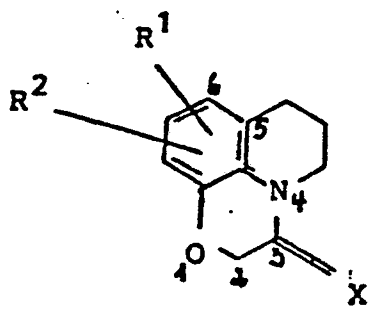

in welcher

X, für Sauerstoff oder Schwefel steht und

$R^1$ und $R^2$ unabhängig voneinander für Wasserstoff, Halogen, Cyano oder Nitro stehen, mit der Einschränkung, daß $R^2$ und $R^1$ nicht gleichzeitig für Wasserstoff stehen, wenn X für Sauerstoff steht,

gefunden.

Weiterhin wurde gefunden, daß man die neuen substituierten Benzoxazin-Derivate der Formel (I)

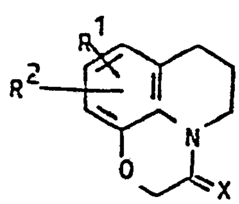

(I)

in welcher

X für Sauerstoff oder Schwefel steht und $R^1$ und $R^2$ unabhangig voneinander fur Wasserstoff, Halogen, Cyano oder Nitro stehen, mit der Einschränkung, daß $R^2$ und $R^1$ nicht gleichzeitig für Wasserstoff stehen, wenn X für Sauerstoff steht,

erhält, wenn man

a) 4,5-Trimethylenbenzoxazinon der Formel (II)

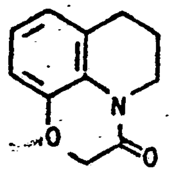

(II)

mit geeigneten elektrophilen Reagenzien, gegebenenfalls in Gegenwart eines Katalysators und gegebenenfalls in Gegenwart eines Verdünnungsmittels direkt substituiert, oder wenn man

b) 8-Hydroxy-1,2,3,4-tetrahydrochinoline der Formel (III)

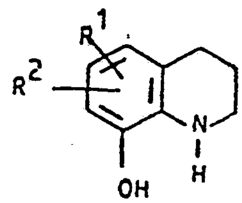

(III)

in welcher
$R^1$ und $R^2$ die oben angegebene Bedeutung haben,
mit einem Halogenacetylhalogenid gegebenenfalls in Gegenwart eines Säurebindemittels und gegebenenfalls in Gegenwart eines Verdünnungsmittels cyclisiert, oder wenn man
c) die nach Verfahren a) oder b) erhaltenen erfindungsgemäßen Benzoxazinone der Formel (Ia)

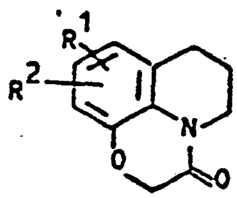

(Ia)

in welcher
$R^1$ und $R^2$ die oben angegebene Bedeutung haben,
mit Phosphorpentasulfid oder anderen Schwefelungsmitteln gegebenenfalls in Gegenwart eines Verdünnungsmittels in die entsprechenden Benzoxazinthione der Formel (Ib)

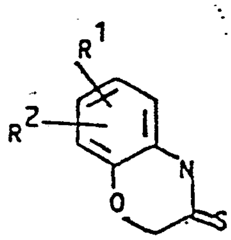

(Ib)

in welcher
$R^1$ und $R^2$ die oben angegebene Bedeutung haben,
überführt, oder wenn man
d) die nach Verfahren a), b) oder c) erhaltenen erfindungsgemäßen Nitrobenzoxazin-Derivate der Formel (Ic)

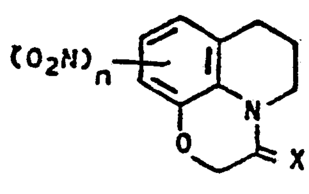

(Ic)

in welcher
X für Sauerstoff oder Schwefel steht und
n für 1 oder 2 steht,
mit Wasserstoff in Gegenwart eines Katalysators und in Gegenwart eines Verdünnungsmittels gegebenenfalls unter Druck zu den entsprechenden Aminoverbindungen der Formel (Id) reduziert,

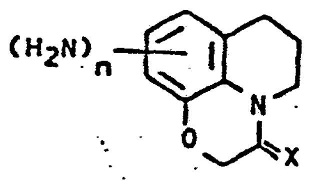

(Id)

in welcher

n und X die oben angegebene Bedeutung haben, und die so erhaltenen Aminobenzoxazin-Derivate der oben angegebenen Formel (Id) mit Natriumnitrit und einer Mineralsäure der Formel HA in Gegenwart eines Verdünnungsmittels diazotiert und die so erhaltenen Diazoniumsalze der Formel (Ie)

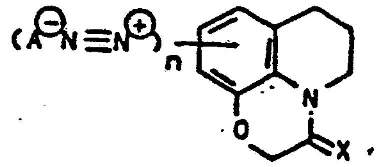

(Ie)

in welcher

n und X die oben angegebene Bedeutung haben und

$A^{\ominus}$ für das Anion einer starken Mineralsäure steht,

mit geeigneten nukleophilen Reagenzien in Gegenwart eines Verdünnungsmittels und gegebenenfalls in Gegenwart eines Katalysators nach der Art der sogenannten Sandmeyer-Reaktion unter Stickstoffabspaltung substituiert, oder die sogenannte Balz-Schiemann-Variante, d.h. thermische Zersetzung der entsprechenden Diazonium-tetrafluoroborat-Verbindung, zur Einführung eines Fluoratoms anwendet. Auch die Einführung der CN-Gruppe läßt sich durch Pyrolyse der trockenen Diazoniumtetracyanocuprate bewerkstelligen.

Es wurde weiterhin die neue Verwendung der substituierten Benzoxazin-Derivate der Formel (I), in der $R^1$, $R^2$ und X die oben angegebene Bedeutung haben, als Fungizide gefunden. Dabei zeigen die erfindungsgemäßen Verbindungen überraschenderweise eine bessere fungizide Wirksamkeit insbesondere bei systemischer Anwendung als die nach dem Stand der Technik bekannten Benzoxazinone (vgl. US-A-4 133 954) Die erfindungsgemäßen Stoffe stellen somit eine Bereicherung der Technik dar.

Die erfindungsgemäßen substituierten Benzoxazin-Derivate sind durch die Formel (I) allgemein definiert.

Im einzelnen seien außer den bei den Herstellungsbeispielen genannten Verbindungen die folgenden Verbindungen der Formel (I) genannt:

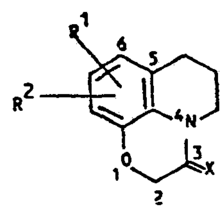

(I)

| $R^1$ | $R^2$ | X |
|---|---|---|
| 6-F | H | O |
| 6-F | H | S |
| 7-F | H | O |
| 7-F | H | S |
| 6-F | 8-F | O |
| 6-F | 8-F | S |

Le A 21 962-Ausland

Vervendet man beispielsweise 4,5-Trimethylen-1,4-benzoxazin-3-on und Chlor als Ausgangsstoffe, so kann der Reaktionsablauf des erfindungsgemäßen Verfahrens a) durch das folgende Formelschema wiedergegeben werden:

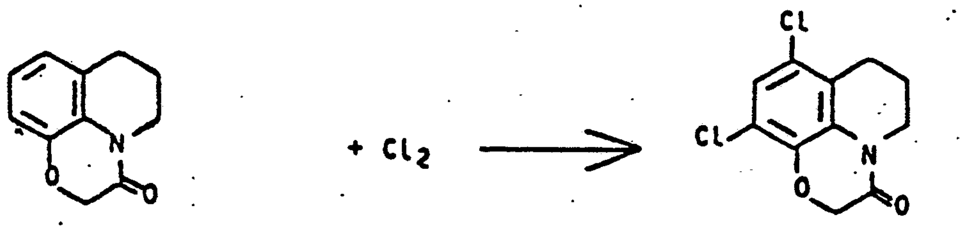

Verwendet man beispielsweise 5-Chlor-8-hydroxy-1,2,3,4-tetrahydrochinolin und Chloracetylchlorid als Ausgangsstoffe, so kann der Reaktionsablauf des erfindungsgemäßen Verfahrens b) durch das folgende Formelschema wiedergegeben werden:

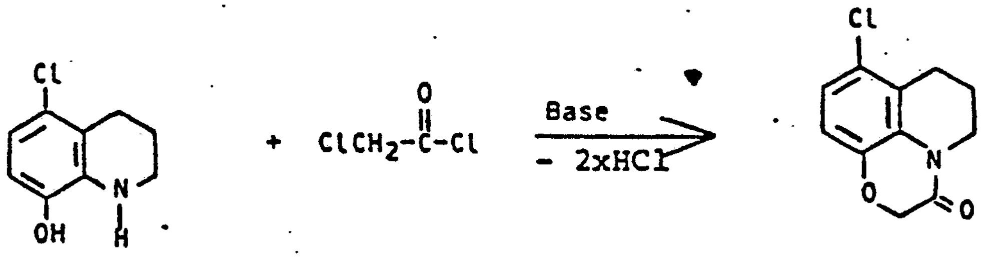


Verwendet man beispielsweise 6-Chlor-4,5-trimethylen-1,4-benzoxazin-3-on und Phosphorpentasulfid als Ausgangsstoffe, so kann der Reaktionsablauf des erfindungsgemäßen Verfahrens c) durch das folgende Formelschema wiedergegeben werden:

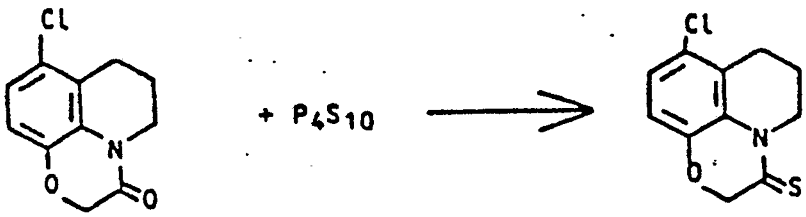

Verwendet man beispielsweise 7-Nitro-4,5-trimethylen-1,4-benzoxazin-3-on und Wasserstoff als Ausgangsstoffe im 1. Reaktionsschritt, so kann der Reaktionsablauf des 1. Schrittes des erfindungsgemäßen Verfahrens d) durch das folgende Formelschema wiedergegegeben werden:

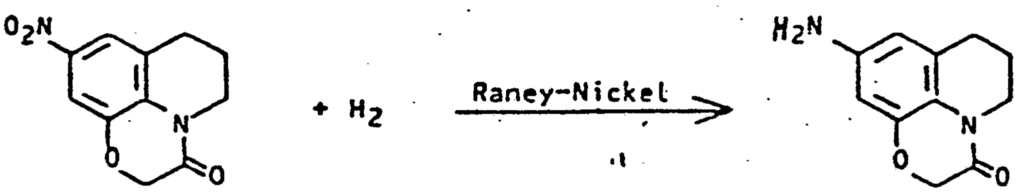


Verwendet man im 2. Reaktionsschritt des Verfahrens d) das so hergestellte 7-Amino-5,6-trimethylen-1,4-benzoxazin-3-on und Natriumnitrit/Salzsäure sowie Kupfercyanid als weitere Reagenzien, so kann der Reaktionsablauf des 2. Schrittes des erfindungsgemäßen Verfahrens d) durch das folgende Formelschema wiedergegeben werden:

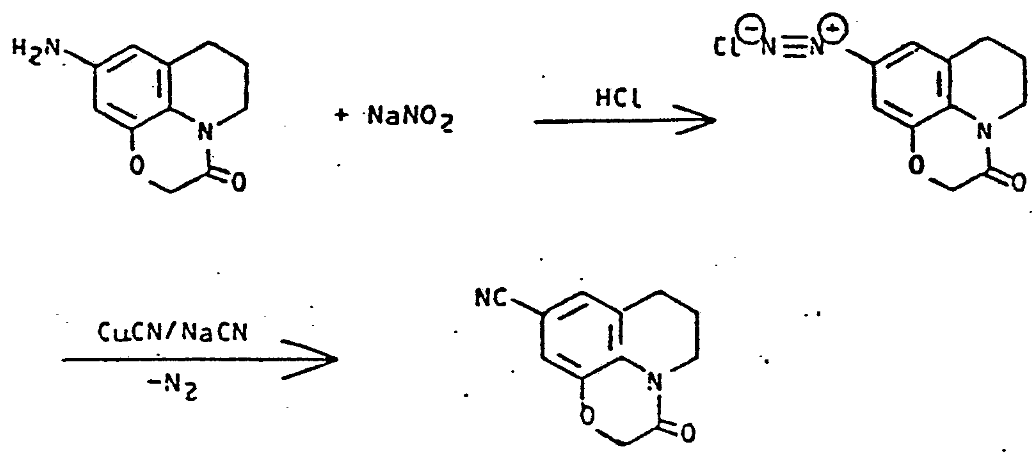


Das als Ausgangsstoff für das erfindungsgemäße Verfahren a) benötigte 4,5-Trimethylenbenzoxazin-3-on der Formel (II) ist bekannt (vgl. US-A-4 133 954).

Die weiterhin als Ausgangsstoffe für das erfindungsgemäße Verfahren a) benötigten elektrophilen Reagenzien sind ebenfalls bekannt.

Bevorzugt verwendet man Halogenierungsmittel, wie z.B. Chlor, Brom oder Interhalogenverbindungen, wie z.B. Bromchlorid oder Jodchlorid, Nitrierungsmittel, wie Salpetersäure.

Es handelt sich hierbei um allgemein bekannte Verbindungen.

Die weiterhin als Ausgangsstoffe für das erfindungsgemäße Verfahren b) benötigten 8-Hydroxy-1,2,3,4-tetrahydrochinoline sind durch die Formel (III) allgemein formuliert.

Die 8-Hydroxy-1,2,3,4-tetrahydrochinoline der Formel (III) sind teilweise bekannt (vgl. z.B. Ind. J. Chem. 12 (1974), 252).

Man erhält sie z.B., indem man die entsprechend substituierten 8-Hydroxychinoline der Formel (IV)

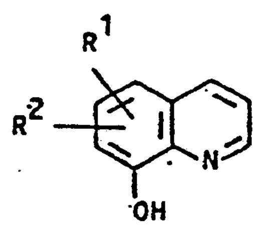

(IV)

in welcher

$R^1$ und $R^2$ die oben angegebene Bedeutung haben,

nach bekannten Verfahren mit Wasserstoff und einem geeigneten Hydrierkatalysator; wie z.B. Raney-Nickel, in einem Verdünnungsmittel, wie z.B. Tetrahydrofuran, bei Temperaturen zwischen 30°C uhd 180°C, gegebenenfalls unter Druck partiell hydriert, oder indem man 8-Hydroxy-1,2,3,4-tetrahydrochinolin der Formel (V)

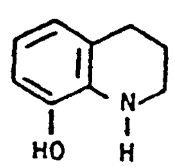

(V)

mit geeigneten elektrophilen Reagenzien in bekannter und üblicher Weise gegebenenfalls in Gegenwart eine Verdünnungsmittels im aromatischen Teil substituiert.

Die substituierten 8-Hydroxychinoline der Formel (IV) sind bekannt (vgl. z.B. J. Amer. Chem. Soc. 66, 1927 (1944)).

Das 8-Hydroxy-1,2,3,4-tetrahydrochinolin der Formel (V) ist ebenfalls bekannt (vgl. Chem. Ber. 14, 1368 (1881).

Die weiterhin als Ausgangsstoffe für das erfindungsgemäße Verfahren d) im 2. Schritt benötigten Mineralsäuren der Formel HA sind ebenfalls allgemein bekannt.

Vorzugsweise verwendet man Chlorwasserstoffsäure, Bromwasserstoffsäure, Schwefelsäure, Salpetersäure, Perchlorsäure, Tetrafluorborsäure.

Die weiterhin als Ausgangsstoffe für das erfindungsgemäße Verfahren d) benötigten Nukleophile sind ebenfalls allgemein bekannt.

Vorzugsweise verwendet man anorganische Cyanide, wie beispielsweise Natrium-, Kalium- oder Kupfercyanid, anorganische Halogenide, wie beispielsweise Natriumoder Kaliumbromid, -chlorid oder -jodid, oder anorganische Tetrafluoroborate, wie z.B. Ammoniumtetrafluoroborat.

Für die erfindungsgemäße Umsetzung nach Verfahren a) kommen als Verdünnungsmittel organische oder wäßrige Lösungsmittel in Frage.

Hierzu gehören insbesondere chlorierte Kohlenwasserstoffe, wie beispielsweise Tetrachlorkohlenstoff, Chloroform oder Dichlormethan.

In einzelnen Fällen kommen auch spezielle Lösungsmittel in Frage, so z.B. im Fall der Nitrierungsreaktion insbesondere Eisessig.

Einige der Reaktionen des erfindungsgemäßen Verfahrens a) werden vorzugsweise in Gegenwart eines geeigneten Katalysators durchgeführt. Für die Nitrierungsreaktionen verwendet man insbesondere Kupfernitrat oder auch Acetanhydrid.

Die Reaktionstemperaturen können bei dem erfindungsgemäßen Verfahren a) in einem weiten Bereich variiert werden. Im Fall der Nitrierungs- und Halogenierungsreaktionen arbeitet man im allgemeinen zwischen -80°C und +80°C, vozugsweise zwischen -40°C und +40°C.

Zur Durchführung des erfindungsgemäßen Verfahrens a) setzt man auf 1 Mol der Benzoxazin-Verbindung der Formel (II) im allgemeinen 1 bis 2 Mol, vorzugsweise 1 bis 1,5 Mol Nitrierungs- oder Halogenierungsmittel ein.

Die Aufarbeitung erfolgt jewwils in bekannter und üblicher Art und Weise.

Für die erfindungsgemäße Umsetzung nach Verfahren b) kommen als Verdünnungsmittel inerte organische Lösungsmittel in Frage. Hiezru gehören insbesondere dioplar-aprotische Solventien, wie beispielsweise Acetonitril oder Propionitril, ferner Amide, wie Dimethylformamid oder Dimethylacetamid oder N-Methylformanilid, und außerdem auch Dimethylsulfoxid, Sulfolan sowie das hochpolare Hexamethylphosphorsäuretriamid.

Das erfindungsgemäß Verfahren b) wird in Gegenwart von Säurebindern vorgenommen. Man kann alls üblicherweise verwendbaren anorganischen und organischen Säurebinder zugeben. Hierzu gehören vorzugsweise Alkalicarbonate, wie beispielweise Natriumcarbonat, Kaliumcarbonat und Natriumhydrogencarbonat, ferner niedere tertiäre Alkylamine, Cycloalkylamine oder Arylalkylamine, wie beispielweise Triethylamin, N,N-Dimethyl-benzylamin, weiterhin Pyridin sowie 1,4-Diazabicyclo (2,2,2)-octan und 1,5-Diazabicyclo (4,3,0)-non-5-en.

Die Reaktionstemperatur kann bei der Durchführung des erfindungsgemäßen Verfahrens b) innerhalb eines größeren Bereichs variiert werden. Im allgemeinen arbeitet man zwischen 0°C und 100°C, vorzugsweise zwischen 10°C und 80°C.

Das erfindungsgemäße Verfahren b) wird im allgemeinen bei Normaldruck durchgeführt.

Zur Durchführung des erfindungsgemäßen Verfahrens b) setzt man auf 1 Mol 8-Hydroxyn 1,2,3,4-tetrahydro-chinolin der Formel (III) im allgemeinen 1,0 bis 1,5 Mol, vorzugsweise 1,0 bis 1,2 Mol Chloracetylchlorid ein.

Die Umsetzung wird vorzugsweise unter Verwendung eines der obengenannten Säurebinder in einem der obengenannten Verdünnungsmittel durchgeführt. Das Reaktionsgemisch wird mehrere Stunden bei der erforderlichen Temperatur gerührt. Die Aufarbeitung der Reaktionsmischung und die Isolierung der erfindungsgemäßen Reaktionsproduke der Formel (I) erfolgt in allgemein üblicher Art und Weise.

Für die erfindungsgemäße Umsetzung nach Verfahren c) kommen als Verdünnungsmittel inerte organische Lösungsmittel in Frage. Hierzu gehören vorzugsweise chlorierte Kohlenwasserstoffe, wie z.B. Tetrachlorkohlenstoff, Chlorbenzol oder Dichlorbenzol.

Die Reaktionstemperaturen können bei der Durchführung des erfindungsgemäßen Verfahrens c) innerhalb eines größeren Bereiches variiert werden. Im allgemeinen arbeitet man zwischen +30°C und +150°C, vorzugsweise bei der Siedetemperatur des verwendeten Lösungsmittels.

Das erfindungsgemäße Verfahren c) wird im allgemeinen bei Normaldruck durchgeführt.

Zur Durchführung des erfindungsgemäßen Verfahrens c) setzt man auf 1 Mol Benzoxazin-Derivat der Formel (Ia) vorzugsweise 0,5 Mol Phosphorpentasulfid ein. Das Reaktionsgemisch wird 10 Minuten bei der erforderlichen Temperatur gerührt, dann vom festen Rückstand abdekantiert und wie üblich aufgearbeitet.

Als Verdünnungsmittel für das erfindungsgemäße Verfahren d) kommen ebenfalls inerte organische Solventien in Frage. Hierzu gehören vorzugsweise aliphatische oder aromatische Kohlenwasserstoffe, wie Benzin, Benzol, Toluol oder Xylol; halogenierte Kohlenwasserstoffe, wie Methylenchlorid, Chloroform, Tetrachlorkohlenstoff oder Chlorbenzol; Ether, wie Diethylether, Dioxan oder Tetrahydrofuran; Ester, wie Ethylacetat; oder Alkohole, wie Methanol, Ethanol oder Isopropanol.

Als Katalysatoren kommen für das erfindungsgemäße Verfahren d) alle üblichen Hydrierkatalysatoren in Frage. Vorzugsweise verwendet man Raney-Katalysatoren, wie z.B. Raney-Nickel oder Edelmetallkatalysatoren, wie z.B. Platin oder Palladium, gegebenenfalls auf einem geeigneten Trägermaterial, wie z.B. Kohle.

Die Reaktionstemperaturen können bei der Durchführung des erfindungsgemäßen Verfahrens d) ebenfalls in einem größeren Bereich variiert werden. Im allgemeinen arbeitet man zwischen +20°C und +120°C, vorzugsweise zwischen +60°C und +100°C.

Das erfindungsgemäße Verfahren d) kann unter erhöhtem Druck durchgeführt werden. Im allgemeinen arbeitet man zwischen 1 und 100 bar, vorzugsweise zwischen 1 und 80 bar.

Bei der Durchführung des erfindungsgemäßen Verfahrens d) setzt man auf 0,1 Mol Nitrobenzoxazinderivat der Formel (Ic) 0,5 bis 5,0 g, vorzugsweise 1 bis 3 g Hydrierkatalysator zu. Die Hydrierung und Aufarbeitung

sowie die Isolierung der Verbindungen der Formel (Id) erfolgt nach bekannten und üblichen Methoden.

Als Verdünnungsmittel für den 2. Schritt des erfindungsgemäßen Verfahrens d) kommt insbesondere Wasser in Frage.

Das erfindungsgemäße Verfahren kann gegebenenfalls in Gegenwart eines geeigneten Katalysators durchgeführt werden. Vorzugsweise verwendet man Kupfer(I)salze, wie z. B. Kupfer-(I)-cyanid oder Kupfer-(I)bromid oder Kupfer-(I)-chlorid.

Die Reaktionstemperaturen können bei der weiteren Durchführung des erfindungsgemäßen Verfahrens ebenfalls in einem größeren Bereich variiert werden. Im allgemeinen arbeitet man zwischen +20°C und +100°C, vorzugsweise zwischen +40°C und +100°C.

Bei der Durchführung des 2. Schrittes des erfindungsgemäßen Verfahrens d) setzt man pro Mol Aminobenzoxazin-Derivat der Formel (Id) im allgemeinen 1 bis 1,5 Mol Natriumnitrit, vorzugsweise 1 bis 1,2 Mol Natriumnitrit, 2 bis 5 Mol Mineralsäure, vorzugsweise 2 bis 3 Mol Mineralsäure sowie 1 bis 5 Mol nukleophiles Reagenz, vorzugsweise 1 bis 3 Mol nukleophiles Reagenz ein.

Die Durchfürung und Aufarbeitung erfolgt in bekannter und üblicher Art und Weise.

Die erfindungsgemäßen Wirkstoffe weisen eine starke mikrobizide Wirkung auf und können zur Bekämpfung von unerwünschten Mikroorganismen praktisch eingesetzt werden. Die Wirkstoffe sind für den Gebrauch als Pflanzenschutzmittel geeignet.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Als Pflanzenschutzmittel können die erfindungsgemäßen Wirkstoffe mit besonders gutem Erfolg zur Bekämpfung von Reiskrankheiten, wie z.B. gegen den Erreger Pyricularia oryzae eingesetzt werden.

Auch die Bekämpfung von Oomyceten und Schorferkrankungen, wie z.B. von Apfelschorf ist möglich.

Die Wirkstoffe können in die üblichen Formulierungen übergeführt werden, wie Lösungen, Emulsionen, Spritzpulver, Suspensionen, Pulver, Stäubemittel, Schäume, Pasten, lösliche Pulver, Granulate, Aerosole, Suspensions-Emulsionskonzentrate, Saatgutpuder, Wirkstoffimprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä. sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkyl-naphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder paraffine, z.B. Erdölfraktionen,

Alkohole, wie Butanol oder Glykol sowie deren Ether und Ester, Ketone, wie Aceton, Methylethylketon, Methylisobutylketon oder Cyclohexanon, starke polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material, wie Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäureester, polyoxyethylen-Fettalkohol-ether, z.B. Alkylarylpolyglykol-ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gew.-% Wirkstoff, vorzugsweise zwischen 0,5 und 90 %.

Die erfindungsgemäßen Wirkstoffe können in den Formulerungen oder in den verschiedenen Anwendungsformen in Mischung mit anderen bekannten Wirkstoffen vorliegen, wie Fungiziden, Bakteriziden, Insektiziden, Akariziden, Nematiziden, Herbiziden, Schutzstoffen gegen Vogelfraß, Wuchsstoffen, Pflanzennährstoffen und Bodenstrukturverbesserungsmitteln.

Die Wirkstoffe können als solche, in Form ihrer Formulierungen oder der daraus durch weiteres Verdünnen bereiteten Anwendungsformen, wie gebrauchsfertige Lösungen, Emulsionen, Suspensionen, Pulver, Pasten und Granulate angewendet werden. Die Anwendung geschieht in üblicher Weise, z.B. durch Gießen, Tauchen, Spritzen, Sprühen, Vernebeln, Verdampfen, Injizieren, Verschlämmen, Verstreichen, Stäuben, Streuen, Trockenbeizen, Feuchtbeizen, Naßbeizen, Schlämmbeizen oder Inkrustieren.

Bei der Behandlung von Pflanzenteilen können die Wirkstoffkonzentrationen in den Anwendungsformen in einem größeren Bereich variiert werden. Sie liegen im allgemeinen zwischen 1 und 0,0001 Gew.-%, vorzugsweise zwischen 0,5 und 0,001 %.

Bei der Saatgutbehandlung werden im allgemeinen Wirkstoffmengen von 0,001 bis 50 g je Kilogram Saatgut, vorzugsweise 0,01 bis 10 g, benötigt.

Bei Behandlung des Bodens sind Wirkstoffkonzentrationen von 0,00001 bis 0,1 gew.-%, vorzugsweise von 0,0001 bis 0,02 Gew.-%, am Wirkungsort erforderlich.

**Herstellungsbeispiele**

**Beispiel 1**

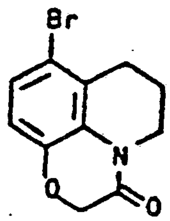

(Verfahren a)

18,9 g (0,1 Mol) 4,5-Trimethylen-1,4-benzoxazin-3-on werden in 250 ml Tetrachlorkohlenstoff gelöst und bei 0° C tropfenweise unter Rühren mit 7,1 g (0,1 Mol) Brom in 50 ml Tetrachlorkohlenstoff versetzt.

Die Reaktionsmischung wird bis zum Ende der HCl-Entwicklung (ca. 6 Stunden) bei 60° C gerührt, nach dem Abkühlen filtriert und im Vakuum eingeengt. Das zurückbleibende Öl kristallisiert beim Verreiben mit Ether. Nach Absaugen und Trocknen erhält man 12 g (53 % der Theorie) an 6-Brom-4,5-trimethylen-1,4-benzoxazin-3-on vom Schmelzpunkt 133°C.

**Beispiel 2**

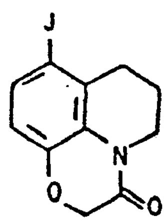

(Verfahren a) Zu einer Lösung von 18,9 g (0,1 Mol) 4,5-Trimethylen-1,4-benzoxazin-3-on in 250 ml Dichlormethan tropft man bei -78° C eine Lösung von 18,6 g (0,1 Mol) Jodchlorid in 10 ml Dichlormethan.

Nach erfolgter Zugabe läßt man die Reaktionsmischung auf Raumtemperatur kommen, entfernt das Lösungsmittel im Vakuum und wäscht den kristallinen Rückstand mit kalten Ethanol. Nach dem Trocknen erhält man 12,7 g (40 % der Theorie) an 6-Jod-4,5-trimethylen-1,4-benzoxazin-3-on vom Schmelzpunkt 191° C.

**Beispiel 3**

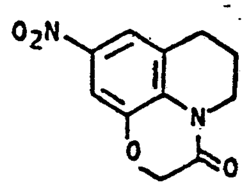

(Verfahren a)

18,9 g (0,1 Mol) 4,5-Trimethylen-1,4-benzoxazin-3-on werden in einem Gemisch aus 200 ml Eisessig und 20 g (0,1 Mol) Acetanhydrid gelöst und bei 10°C tropfenweise unter Rühren mit 10 g (0,1 Mol) 66 %iger Salpetersäure versetzt.

Die Reaktionsmischung wird 2 Stunden bei 20°C bis 25°C gerührt, dann mit 100 ml Wasser versetzt und mit insgesamt 600 ml Dichlormetham mehrfach extrahiert. Die vereinigten organischen Extrakte werden über Natriumsulfat getrocknet und im Vakuum eingeengt. Das zurückbleibende braune Öl kristallisiert beim Verreiben mit Ether in gelben Kristallen. Man erhält 19 g (80 % der Theorie) an 7-Nitro-4,5-trimethylen-1,4-benzoxazin-3-on vom Schmelzpunkt 103 bis 105°C.

**Beispiel 4**

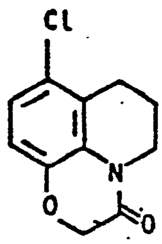

(Verfahren b)

22 g (0,1 Mol) 5-Chlor-8-hydroxy-1,2,3,4-tetrahydro-chinolinhydrochlorid, 11,3 g (0 1 Mol) Chloracetylchlorid und 41,4 g (0,3 Mol) Kaliumcarbonat werden in 300 ml Acetonitril 5 Stunden am Rückfluß gekocht.

Man läßt die Reaktionsmischung abkühlen, filtriert und engt das Filtrat im Vakuum ein, wobei das Produkt auskristallisiert. Man erhält 19 g (85 % der Theorie) an 6-Chlor-4,5-trimethylen-1,4-benzoxazin-3-on vom Schmelzpunkt 98°C.

**Herstellung des Ausgangsproduktes**

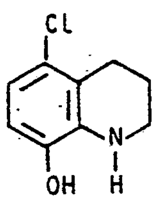

(III-1)

Zu 14,g g (0,1 Mol) 8-Hydroxy-1,2,3,4-tetrahydro-chinolin in 100 ml Dichlormethan leitet man bei -78°C 7,1 g (0,1 Mol) Chlor ein.

Man läßt den Ansatz auf Raumtemperatur kommen, rührt 10 Minuten mit Aktivkohle, filtriert und engt im Vakuum ein. Man erhält so 16,5 g (75 % der Theorie) an 5°Chlor-8-hydroxy-1,2,3,4-tetrahydrochinolin.

In analoger Weise erhält man mit 11,5 g (0,1 Mol) Bromchlorid statt Chlor 14,5 g (55 % der Theorie) an 5-Brom-8-hydroxy-1,2,3,4-tetrahydrochinolin (III-2).

**Beispiel 5**

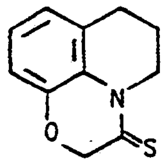

(Verfahren c)

Zu 18,9 g (D,1 Mol) 4,5-Trimethylen-1,4-benzoxazin-3-on in 200 ml Chlorbenzol gibt man unter Rühren portionsweise 11,1 g (0,05 Mol) Phosphorpentasulfid.

Man erhitzt 10 Minuten auf Rückflußtemperatur, läßt die Mischung abkühlen und dekantiert vom ungelösten Rückstand ab. Durch Abdampfen des Lösungsmittels erhält man 19,5 g (95 % der Theorie) an 4,5-Trimethylen-1,4-Benzoxazin-3-thion vom Schmelzpunkt 103° C.

**Beispiel 6**

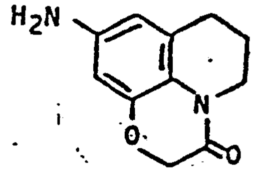

(Verfahren d) 1. Schritt

5,8 g (0,025 Mol) 7-Nitro-4,5-trimethylen-1,4-benzoxazin-3-on werden in 50 ml Tetrahydrofuran gelöst, mit 1 g Raney-Nickel versetzt und 2 Stunden mit Wasserstoff unter Druck (80 bar) auf 60° C erhitzt.

Nach beendeter Reaktion wird der Katalysator abfiltriert und das Lösungsmittel im Vakuum eingedampft. Der zurückbleibende Kristallbrei wird mit 100 ml Ether ausgekocht, nach dem Abkühlen abgesaugt und getrocknet. Man erhält 3,3 g (64 % der Theorie) an 7-Amino-4,5-trimethylen-1,4-benzoxazin-3-on vom Schmelzpunkt 170° C.

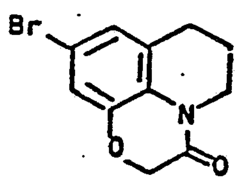

(Verfahren d) 2. Schritt

20,4 g (0,1 Mol) 7-Amino-4,5-trimethylen-1,4-benz-oxazin-3-on werden in 50 ml (0,3 Mol) 50 %iger Bromwasserstoffsäure mit 6,9 g (0,1 Mol) Natriumnitrit versetzt.

Die Reaktionslösung wird bei 0° C zu einer Lösung von 14,3 g (0,1 Mol) Kupfer-(I)-bromid und 10,3 g (0,1 Mol) Natriumbromid in 50 ml Wasser getropft. Man erwärmt bis zum Ende der Gasentwicklung. Nach dem Abkühlen extrahiert man mehrmals mit Dichlormethan, trocknet die vereinigten organischen Extrakte über Natriumsulfat, entfernt das Lösungsmittel im Vakuum und kristallisiert den so erhaltenen Feststoff aus Cyclohexan um. Man erhält 13,4 g (50 % der Theorie) an 7-Brom-4,5-trimethylen-1,4-benzoxazin-3-on in Form gelber Kristalle vom Schmelzpunkt 168° C.

In entsprechender Weise erhält man die folgenden Verbindungen der allgemeinen Formel (I):

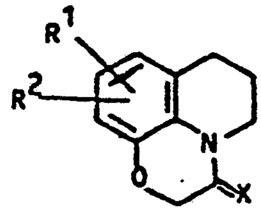

(I)

| Beisp.-Nr. | | Physikalische Eigenschaften |
|---|---|---|
| 7 | 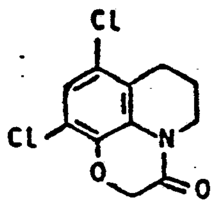 | Fp. 152°C-156°C |
| 8 | 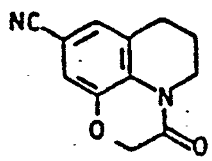 | IR : 2.200 $cm^{-1}$ (CN-Bande)<br>Fp. 147°-149°C |
| 9 | 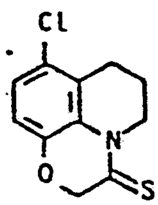 | Fp. 104°C |
| 10 | 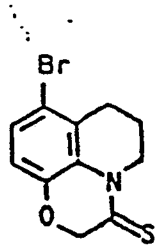 | Fp. 132°C |

| Beisp.-Nr. | | Physikalische Eigenschaften |
|---|---|---|
| 11 | 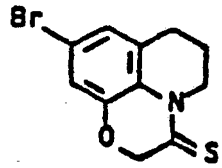 | Fp. 169°C-172°C |
| 12 | 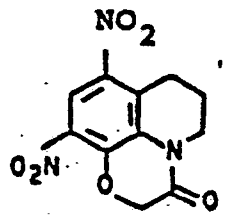 | Fp. 142°C-146°C |
| 13 | 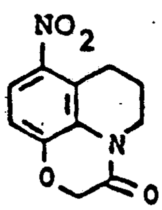 | Fp. 162°C-168°C |

| Beispiel Nr. | | Physikalische Eigenschaften |
|---|---|---|
| 14 | 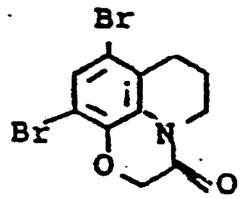 | Fp. 193°C-194°C |
| 15 | 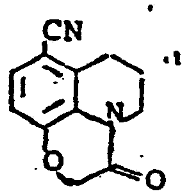 | Fp. 191°C-193°C |
| 16 | 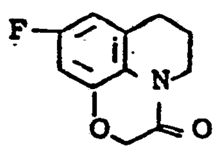 | Fp. 90°C-110°C |
| 17 | 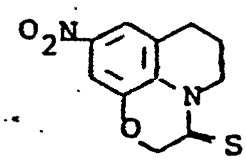 | Fp. 169°C-173°C |
| 18 | 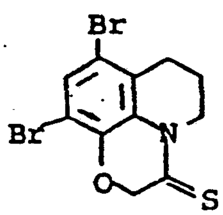 | Öl |

**Anwendungsbeispiele**

Bei den nachstehenden Anwendungsbeispielen wird die hier angegebene Substanz als Vergleichsbeispiel herangezogen:

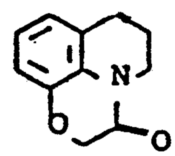

4,5-Trimethylen-1,4-benzoxazin-3-on

**Beispiel A**

Pyricularia-Test (Reis) / systemisch
Lösungsmittel: 12,5 Gew.-Teile Aceton
Emulgator: 0,3 Gew.-Teil Alkylarylpolyglykolether

Zur Herstellung einer zweckmäßigen Wirkstoffzubereitung vermischt man 1 Gew.-Teil Wirkstoff mit der angegebenen Menge Lösungsmittel und verdünnt das Konzentrat mit Wasser und der angegebenen Menge Emulgator auf die gewünschte Konzentration.

Zur Prüfung auf systemische Eigenschaften werden 40 ml der Wirkstoffzubereitung auf Einheitserde gegossen, in der junge Reispflanzen angezogen wurden. 7 Tage nach der Behandlung werden die Pflanzen mit einer wäßrigen Sporensuspension von Pyricularia oryzae inokuliert. Danach verbleiben die Pflanzen in einem Gewächshaus bei einer Temperatur von 25° C und einer rel. Luftfeuchtigkeit von 100 % bis zur Auswertung.

4 Tage nach der Inokulation erfolgt die Auswertung des Krankheitsbefalls.

Eine deutliche Überlegenheit in der Wirksamkeit gegenüber dem Stand der Technik zeigen bei diesem Test z.B. die Verbindungen gemäß folgender Herstellungsbeispiele: 1, 3, 4.

**Beispiel B**

Pyricularia-Test (Reis) / protektiv
Lösungsmittel: 12,5 Gew.-Teile Aceton
Emulgator: 0,3 Gew.-Teile Alkylarylpolyglykolether

Zur Herstellung einer zweckmäßigen Wirkstoffzubereitung vermischt man 1 Gew.-Teil Wirkstoff mit der angegebenen Menge Lösungsmittel und verdünnt das Konzentrat mit Wasser und der angegebenen Menge Emulgator auf die gewünschte Konzentration.

Zur Prüfung auf protektive Wirksamkeit bespritzt man Junge Reispflanzen mit der Wirkstoffzubereitung bis zur Tropfnässe. Nach dem Abtrocknen des Spritzbelages werden die Pflanzen mit einer wäßrigen Sporensuspension von Pyricularia oryzae inokuliert. Anschließend werden die pflanzen in einem Gewächshaus bei 100 % rel. Luftfeuchtigkeit und 25° C aufgestellt.

4 Tage nach der Inokulation erfolgt die Auswertung des Krankheitsbefalls.

Eine gute Wirksamkeit zeigen bei diesem Test z.B. die Verbindungen gemäß folgender Herstellungsbeispiele: 1, 3, 4.

**Patentansprüche**

1. Substituierte Benzoxazin-Derivate der Formel

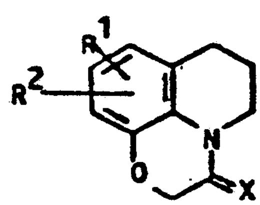

(I)

in welcher
X für Sauerstoff oder Schwefel steht und
$R^1$ und $R^2$ unabhängig voneinander für Wasserstoff, Halogen, Cyano oder Nitro stehen, mit der Enschränkung, daß $R^2$ und $R^1$ nicht gleichzeitig für Wasserstoff stehen, wenn X für Sauerstoff steht.

2. Verfahren zur Herstellung von substituierten Benzoxazin-Derivaten der Formel

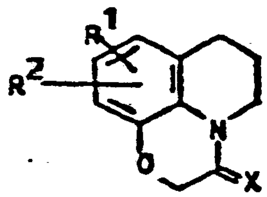

(I)

in welcher
X für Sauerstoff oder Schwefel steht und
$R^1$ umd $R^2$ unabhängig voneinander für Wasserstoff, Halogen, Cyano oder Nitro stehen, mit der Einschränkung, daß $R^2$ und. $R^1$ nicht gleichzeitig für Wasserstoff stehen, wenn X für Sauerstoff steht,
dadurch gekennzeichnet, daß man
a) 4,5-Trimethylenbenzoxazinon der Formel (II)

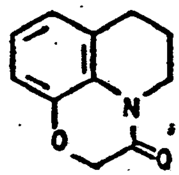

(II)

mit geeigneten elektrophilen Reagenzien gegebenenfalls in Gegenwart eines Katalysators und gegebenenfalls in Gegenwart eines Verdünnungsmittels substituiert, oder
b) 8-Hydroxy-1,2,3,4-tetrahydrochinoline der Formel (III),

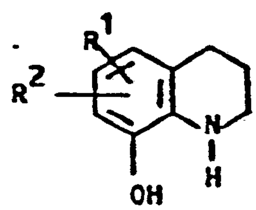

(III)

in welcher
$R^1$ und $R^2$ die oben angegebene Bedeutung haben,
mit einem Halogenacetylhalogenid gegebenenfalls in Gegenwart eines Säurebindemittels und gegebenenfalls in Gegenwart eines Verdünnungsmittels cyclisiert, oder
c) die nach Verfahren a) oder b) erhaltenen erfindungsgemäßen Benzoxazinone der Formel (Ia)

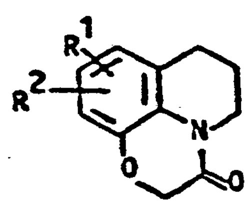

(Ia)

in welcher
$R^1$ und $R^2$ die oben angegebene Bedeutung haben,
mit phosphorpentasulfid oder anderen Schwefelungsmitteln gegebenenfalls in Gegenwart eines Verdünnungsmittels in die entsprechenden Benzoxazinthione der Formel (Ib)

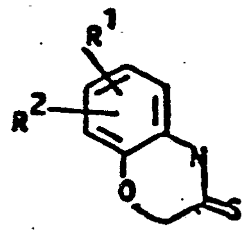

(Ib)

in welcher
$R^1$ und $R^2$ die oben angegebene Bedeutung haben,
überführt, oder
d) die nach Verfahren a), b) oder c) erhaltenen erfindungsgemäßen Nitrobenzoxazin-Derivate der Formel (Ic)

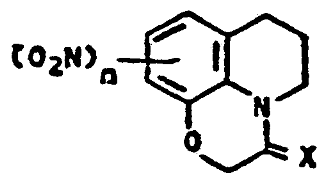

(Ic)

in welcher
X für Sauerstoff oder Schwefel steht und
n für 1 oder 2 steht,
mit Wasserstoff in Gegenwart eines Katalysators und in Gegenwart eines Verdünnungsmittels gegebenenfalls unter Druck zu den entsprechenden Amminoverbindungen der Formel (Id) reduziert,

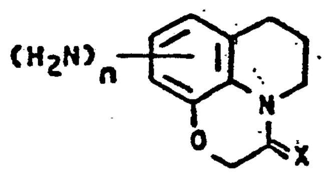

(Id)

in welcher
n und X die oben angegebene Bedeutung haben,
die so erhaltenen Aminobenzoxazin-Derivate der oben angegebenen Formel (Id) mit Natriumnitrit und einer Mineralsäure in Gegenwart eines Verdünnungsmittels diazotiert und die so erhaltenen Diazoniumsalze der Formel (Ie),

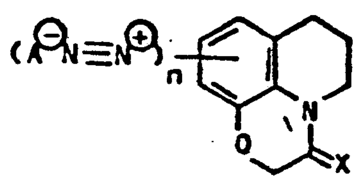

(Ie)

in welcher
n und X die oben angegebene Bedeutung haben und
$A^{\ominus}$ für das Anion einer starken Mineralsäure steht,
mit geeigneten nukleophilen Reagenzien in Gegenwart eines Verdünnungsmittels und gegebenenfalls in Gegenwart eines Katalysators in Form einer Sandmeyer-Reaktion oder nach der Balz-Schiemann-Variante umsetzt.

3. Schädlingsbekämpfungsmittel, gekennzeichnet durch einen Gehalt an mindestens einem substituierten Benzoxazin-Derivat der Formel (I) gemäß Anspruch 1.

4. Verwendung von substituierten Benzoxazin-Derivaten der Formel (I) gemäß Anspruch 1 zur Bekämpfung von Schädlingen.

5. Verfahren zur Bekämpfung von Schädlingen, dadurch gekennzeichnet, daß man substituierte Benzoxazin-Derivate der Formel (I) gemäß Anspruch 1 auf Schädlinge und/oder ihren Lebensraum einwirken läßt.

6. Verfahren zur Herstellung von Schädlingsbekämpfungsmitteln, dadurch gekennzeichnet, daß man substituierte Benzoxazin-Derivate der Formel (I) gemäß Anspruch 1 mit Streckmitteln und/oder oberflächenaktiven Mitteln vermischt.

**Claims**

1. Substituted benzoxazine derivatives of the formula

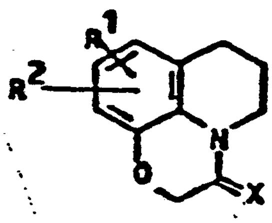

(I)

in which

X represents oxygen or sulphur and

$R^1$ and $R^2$ independently of one another represent hydrogen, halogen, cyano or nitro, with the restriction that $R^2$ and $R^1$ do not simultaneously represent hydrogen when X represents oxygen.

2. Process for the preparation of substituted benzoxazine derivatives of the formula

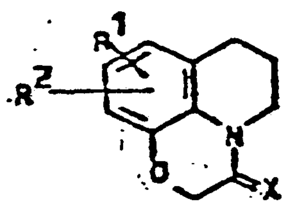

(I)

in which

X represents oxygen or sulphur and

$R^1$ and $R^2$ independently of one another represent hydrogen, halogen, cyano or nitro, with the restriction that $R^2$ and $R^1$ do not simultaneously represent hydrogen when X represents oxygen,

characterised in that

a) 4,5-trimethylenebenzoxazinone of the formula (II)

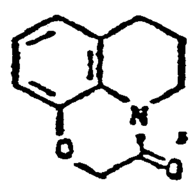

(II)

is substituted with suitable electrophilic reagents, if appropriate in the presence of a catalyst and if appropriate in the presence of a diluent, or b) 8-hydroxy-1,2,3,4-tetrahydroquinolines of the formula (III)

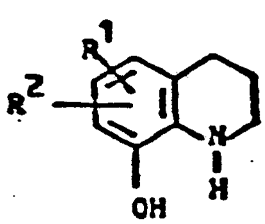

(III)

in which $R^1$ and $R^2$ have the abovementioned meaning, are cyclised with a halogenoacetyl halide, if appropriate in the presence of an acid-binding agent and if appropriate in the presence of a diluent, or

c) the benoxazinones according to the invention obtained by process a) or b), of the formula (Ia)

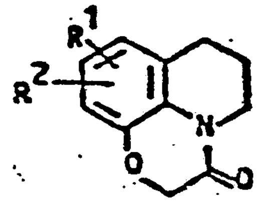

(Ia)

in which

$R^1$ and $R^2$ have the abovementionned meaning, are converted into the corresponding benzoxazine-thiones of the formulas (Ib)

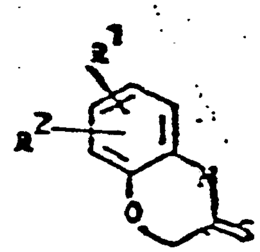

(Ib)

in which

$R^1$ and $R^2$ have the abovementioned meaning, with phosphorus pentasulphide or other sulphurising agents, if appropriate in the presence of a diluent, or

d) the nitrobenzoxamine derivatives according to the invention obtained by process a), b) or c), of the formula (Ic)

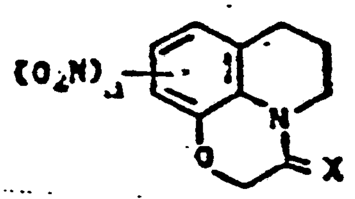

(Ic)

in which

X represents oxygenor sulphur and

n represents 1 or 2,

are reduced with hydrogen in the presence of a catalyst and in the presence of a diluent, if appropriate under pressure, to give the corresponding amino compounds of the formula (Id)

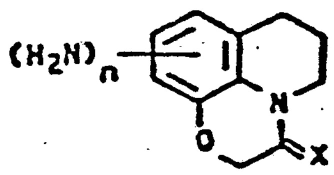

(Id)

in which

n and X have the abovementioned meaning, the aminobenzoxazine derivatives thus obtained, of the abovementioned formula (Id), are diazotised with sodium nitrite and a mineral acid in the presence of a diluent, and the diazonium salts thus obtained, of the formula (Ie)

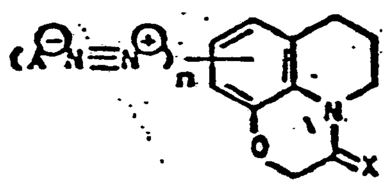

(Ie)

in which
n and X have the abovementioned meaning and
$A^{\theta}$ represents the anion of a strong mineral acid, are reacted with suitable nucleophilic reagents in the presence of a diluent and if appropriate in the presence of a catalyst, in the form of a Sandmeyer reaction or by the Balz-Schiemann variant.

3. Agents for combating pests characterised in that they contain at least one substituted benzoxazine derivative of the formula (I) according to Claim 1.

4. Use of substituted benzoxazine derivatives of the formula (I) according to Claim 1 for combating pests.

5. Process for combating pests, characterised in that substituted benzoxazine derivatives of the formula (I) according to Claim 1 are allowed to react on pests and/or their environment.

6. Process for the preparation of agents for combating pest , characterised in that substituted benzoxazine derivatives of the formula (I) according to Claim 1 are mixed with extenders and/or surface-active agents.

**Revendications**

1. Dérivés substitués de benzoxazine de formule:

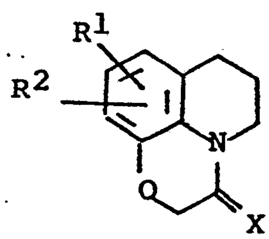

(I)

dans laquelle
X représente de l'oxygéne ou du soufre et
$R^1$ et $R^2$ indépendamment l'un de l'autre représentent de l'hydrogène, de l'halogène, cyano ou nitro, avec la restriction que $R^2$ et $R^1$ ne représentent pas simultanément de l'hydrogène lorsque X représente de l'oxygène.

2. Procédé de fabrication de dérivés substitués de benzoxazine de formule

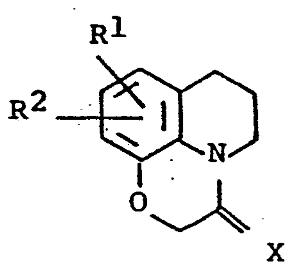

(I)

dans laquelle
X représente de l'oxygène ou du soufre et
$R^1$ et $R^2$ indépendamment l'un de l'autre représentent de l'hydrogène, de l'halogène, cyano ou nitro, avec la restriction que $R^2$ et $R^1$ ne représentent pas simultanément de l'hydrogène lorsque X signifie de l'oxygène,
caractérisé en ce que
a) on substitue la 4,5-triméthyléne-benzoxazinone de formule (II)

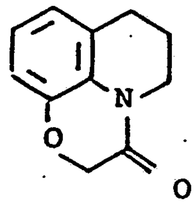

(II)

avec des réactifs électrophiles appropriés, éventuellement en présence d'un catalyseur et éventuellement en présence d'un diluant, ou

b) on cyclise des 8-hydroxy-1,2,3,4-tétrahydroquinoléines de formule (III)

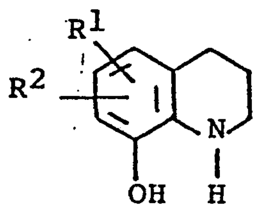

(III)

dans laquelle

$R^1$ et $R^2$ ont la signification indiquée plus haut,

avec un halogénure d'halogénoacétyle éventuellement en présence d'un agent fixateur d'acide et éventuellement en présence d'un diluant, ou

c) on convertit les benzoxazinones conformes à l'invention de formule (Ia) obtenues par le procédé a) ou b):

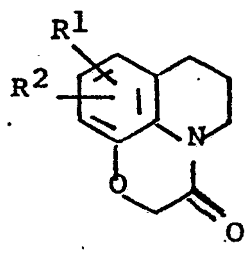

(Ia)

dans laquelle

$R^1$ et $R^2$ ont la signification indiquée plus haut,

avec du pentasulfure de phosphore ou d'autres agents de sulfuration, éventuellement en présence d'un diluant, en les benzoxazinethiones correspondantes de formule (Ib):

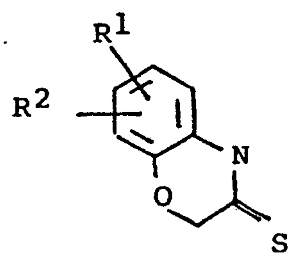

(Ib)

dans laquelle

$R^1$ et $R^2$ ont la signification indiquée plus haut, ou

d) on réduit les dérivés de nitrobenzoxazine conformes à l'invention obtenus par le procédé a), b) ou c), de formule (Ic):

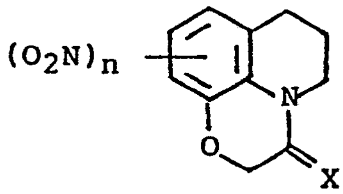

(Ic)

dans laquelle

X représente de l'oxygâne ou du soufre et

n représente 1 ou 2,

avec de l'hydrogène en présence d'un catalyseur et en présence d'un diluant, éventuellement sous pression, en les aminocomposés correspondants de formule (Id):

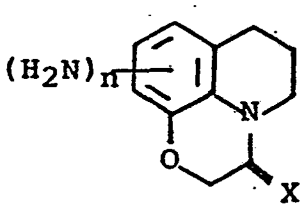

(Id)

dans laquelle

n et X ont la signification indiquée plus haut,

on diazote les dérivés d'aminobenzoxazine ainsi obtenus de formule (Id) indiquée ci-dessus avec du nitrite de sodium et un acide minéral en présence d'un diluant et l'on fait réagir les sels de diazonium ainsi obtenus de formule Ie):

(Ie)

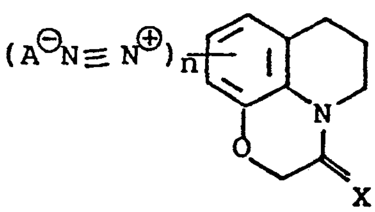

dans laquelle

n et X ont la signification indiquée plus haut et

$A^{\theta}$ représente l'anion d'un acide minéral fort, avec des réactifs nucléophiles appropriés en présence d'un diluant et éventuellement en présence d'un catalyseur sous la forme d'une réaction de Sandmeyer ou selon la variante de Balz-Schiemann.

3. Agents antiparasitaires, caractérisés par une teneur en au moins un dérivé substitué de benzoxazine de formule (I) selon la revendication 1.

4. Utilisation de dérivés substitués de benzoxazine de formule (I) selon la revendication 1 pour combattre les parasites.

5. Procédé pour combattre les parasites, caractérisé en ce que l'on fait agir des dérivés substitués de benzoxazine de formule (I) selon la revendication 1 sur les parasites et/ou sur leur espace vital.

6. Procédé de fabrication d'agents antiparasitaires, caractérisé en ce qu'on mélange des dérivés substitués de benzoxazine de formule (I) selon la revendication 1 avec des diluants et/ou des agents tensioactifs.